# FASCICULE DE BREVET EUROPEEN

(11) **EP 4 447 861 B1**
(45) Date de publication et mention de la délivrance du brevet: **20.05.2026**
(21) Numéro de dépôt: 22839210.6
(22) Date de dépôt: 14.12.2022
(51) Int. Cl.: A61F 2/08, A61L 27/28, A61L 27/30, A61L 27/32, A61L 27/34, A61L 27/54, A61L 27/56, A61L 27/58, A61L 27/18

(54) **DISPOSITIF IMPLANTABLE À LIBERATION PROGRESSIVE D'UN OU PLUSIEURS AGENT(S) FONCTIONNEL(S) ET PROCÉDÉ DE FABRICATION D'UN TEL DISPOSITIF**
IMPLANTIERBARE VORRICHTUNG MIT PROGRESSIVER FREISETZUNG EINES ODER MEHRERER FUNKTIONELLER WIRKSTOFFE UND VERFAHREN ZUR HERSTELLUNG SOLCH EINER VORRICHTUNG
IMPLANTABLE DEVICE WITH GRADUAL RELEASE OF ONE OR MORE FUNCTIONAL AGENTS AND METHOD FOR MANUFACTURING SUCH A DEVICE

(30) Priorité: 15.12.2021 FR 2113580
(43) Date de publication de la demande: 23.10.2024
(73) Titulaire: Cousin Biotech, 59117 Wervicq Sud (FR)
(72) Inventeur: NOEL, Stéphane, 59496 Hantay (FR)
(74) Mandataire: Cabinet Beau de Loménie
(86) Numéro de dépôt international: PCT/EP2022/085949
(87) Numéro de publication internationale: WO 2023/111075

(56) Documents cités:
- EP-A2- 0 452 807
- WO-A1-2020/194280
- IT-A1- 201800 002 536
- US-A- 3 987 497
- US-A- 5 217 495
- US-A1- 2013 183 352
- US-B2- 10 653 819

## Description

### Domaine Technique

La présente invention concerne le domaine technique des dispositifs implantables à libération progressive d'un ou plusieurs agent(s) fonctionnel(s), notamment d'un agent ostéo-inducteur et/ou ostéo-conducteur, en particulier pour la reconstruction d'un ligament croisé antérieur.

### Technique antérieure

La détérioration, notamment la rupture du ligament croisé antérieur, en particulier celui du genou, est fréquente, notamment s'agissant des personnes pratiquant une activité sportive. Ce type de blessure affecterait d'après des études environ 100 000 à 200 000 des athlètes aux USA par an. En sus d'une instabilité articulaire, les signes cliniques peuvent se manifester par des douleurs et un gonflement post-traumatique.

Anatomiquement, le ligament croisé antérieur (LCA) est une structure qui connecte le fémur au tibia, et assure différents mouvements dans la direction antérieure et inhibe la rotation extrême du genou.

Les ligaments sont connus pour ne pas se régénérer et présenter une vascularisation limitée. Les LCA ont ainsi une pauvre capacité à guérir par eux-mêmes.

Environ au moins 150 000 interventions chirurgicales sont effectuées chaque année aux USA pour réparer une anomalie du LCA, et permettre à ce dernier d'assurer ses fonctions normales. Généralement, les stratégies de réparation et de restauration des fonctions du LCA sont basées sur des autogreffes ou des allogreffes, telle que l'autogreffe du tendon de Hamstring et l'autogreffe osseuse du tendon rotulien. La technique chirurgicale consiste à introduire un greffon dans un tunnel foré dans un élément osseux et de fixer ce greffon entre le tibia et le fémur avec des organes de fixation spécifiques, telles que des fixations par vis d'interférence. Ce type d'intervention nécessite souvent une intervention chirurgicale de révision ultérieure. En sus des risques de comorbidité (infection, rejet,...) associés avec l'utilisation de greffons biologiques, la reconstruction du LCA peut échouer du fait de certains problèmes, tels que le toit inter-condylien, les impacts du greffon, le placement dans un tunnel non anatomique ou encore l'incapacité à former une zone de transition à l'interface du greffon et de l'os.

Une guérison incomplète du ligament peut compromettre le succès d'une procédure de reconstruction par manque d'ostéo-intégration. Ce manque d'ostéo-intégration toucherait environ 15% à 25% des patients traités par chirurgie.

Les propriétés histologiques et biomécaniques du LCA sont assurées par l'anthèse, ce site d'attache présent dans le LCA natif est composé de trois régions : le tissu fibro-cartilagineux, le ligament et l'os. La reproduction de ces structures joue un rôle important dans le succès d'une reconstruction d'un ligament, en particulier du LCA.

Des dispositifs pour la reconstruction de ligaments ou tendons sont connus des documents IT-A-2018 0000 2536 et EP-A-0 452 807.

Il existe ainsi un besoin pour des structures implantables d'ingénierie tissulaire interfaciale. Récemment, les structures d'ingénierie tissulaire ont reçu un intérêt croissant et sont considérées comme une alternative prometteuse pour développer une approche thérapeutique qui traite des défauts osseux et de la restauration des tissus vivants en utilisant des biomatériaux, des cellules et des facteurs de croissance. Essayer de recréer la morphologie osseuse est un des sujets de recherche les plus complexes à cause de la complexité de cette structure osseuse et sa propriété de remodelage et de modification perpétuelle de sa forme. Histologiquement, l'os est fait de cellules vivantes et d'une matrice extracellulaire secrétée par des ostéoblastes. La matrice est composée de deux phases, la première phase comprenant des éléments organiques qui contiennent essentiellement du collagène de type I (à 90%) et des protéines non collagéniques (à 10%). Cette première phase est responsable de la flexibilité osseuse et de la résistance à la torsion et en tension. La seconde phase est la phase minéralisée constituée par 60% du volume de la matrice extracellulaire et contient de l'hydroxyapatite (Ca₁₀(PO₄)₆(OH)₁₂) disposé en cristaux, assurant la dureté de l'os.

Le dispositif implantable pour l'ingénierie tissulaire osseuse doit être biocompatible, éventuellement biodégradable intégralement ou en partie, favoriser l'ostéo-intégration et être suffisamment résistant mécaniquement pour supporter les charges en tension, et/ou en rotation, subit par un tendon ou un ligament.

Par ailleurs, la structure dans un matériau synthétique hydrophobe des dispositifs implantables, par exemple en polyéthylène téréphtalate, a tendance à inhiber l'adhésion tissulaire.

Il existe donc un besoin pour un dispositif implantable d'ingénierie tissulaire osseuse résolvant les problèmes précités.

### Exposé de l'invention

La présente invention a pour objet avantageusement, un dispositif implantable pour l'ingénierie tissulaire osseuse, en particulier dans la reconstruction d'un ligament ou d'un tendon en liaison avec un élément osseux ayant une structure qui reproduit/imite la matrice extracellulaire osseuse et favorise la formation d'os, c'est-à-dire favorise l'ostéo-conduction.

La présente invention a pour objet avantageusement un dispositif implantable favorisant l'ostéo-intégration, imitant le comportement d'un ligament ou d'un tendon mécaniquement, et favorisant l'adhésion des cellules.

La présente invention concerne un dispositif et un procédé tels que définis dans les revendications ci-jointes.

La présente invention a pour objet, selon un premier aspect, un dispositif implantable, palliant tout ou partie des problèmes précités, en particulier pour la reconstruction d'un ligament croisé antérieur ou d'un tendon, comprenant une âme longiligne et un membre de couverture longiligne creux comprenant un volume intérieur recevant au moins en partie ladite âme longiligne. Avantageusement, ladite âme longiligne comprend une matrice polymère bio-érodable en milieu aqueux et un ou plusieurs agent(s) fonctionnel(s), et le membre de couverture comprend des orifices traversants en communication fluidique avec ledit volume intérieur.

Avantageusement, la matrice polymère bio-érodable est disposée dans le volume intérieur du membre creux, de sorte que sa désintégration progressive, en particulier sa solubilisation progressive, est ralentie par la structure du membre creux longiligne.

De plus, la combinaison de l'âme longiligne et du membre creux longiligne permettent d'apporter les propriétés de résistance mécanique en tension et en torsion recherchées pour reproduire les propriétés d'un tendon ou d'un ligament.

Avantageusement, le membre creux longiligne permet également d'immobiliser le ou les agent(s) fonctionnel(s) dans le volume intérieur de manière contrôlée selon l'arrangement des orifices traversants retenu.

Cette disposition particulière du dispositif permet de contrôler la libération progressive du ou des agent(s) fonctionnel(s).

La libération progressive est ainsi contrôlée d'une part par le caractère bio-érodable en milieu aqueux de la matrice mais également par la barrière mécanique du membre creux dont la structure est déterminée pour permettre d'une part la libération du ou des agent(s) fonctionnel(s) à travers ses orifices mais également pour permettre la formation d'os selon sa surface extérieure.

On comprend par dispositif implantable dans le présent texte tout dispositif configuré pour pouvoir être implanté dans un corps humain ou animal, pendant une période de plusieurs jours à plusieurs mois ou plusieurs années.

### Matrice polymère bio-érodable

On comprend dans le présent texte par bio-érodable, la dégradation, le désassemblage, ou la digestion de la matrice polymère, et notamment du ou des polymère(s) bio-résorbable(s) que la matrice comprend, par l'action d'un ou plusieurs facteur(s) biologique(s) environnemental(aux) (par exemple l'acidité, la température, ou l'humidité du site ciblé, l'existence d'enzyme(s), de protéine(s) ou d'autre(s) molécule(s) sur le site ciblé) ou par l'action des propriétés physiques ou chimiques du ou des agent(s) fonctionnel(s) dispersé(s) dans la matrice, de préférence par l'action au moins d'un milieu aqueux, encore de préférence par l'action de l'eau contenue dans le milieu du site d'implantation.

On comprend dans le présent texte par matrice polymère, une structure physique de polymère(s) qui retient/retiennent un ou des agent(s) fonctionnel(s).

Avantageusement, la matrice polymère comprend un ou plusieurs polymère(s) bio-résorbable(s), notamment a une structure déterminée solide avant sa mise en contact avec un milieu aqueux.

De préférence, on comprend dans le présent texte par le terme « polymère » : un polymère ou un oligomère comprenant un motif de répétition ou deux ou trois ou plus motifs de répétition différents entre-eux.

On désigne ainsi dans le présente texte par le terme copolymère, tout polymère ou oligomère comprenant deux, ou plus, motifs de répétition différents entre-eux.

En particulier, dans le présent texte, un oligomère comprend un nombre de motifs de répétition inférieur ou égal à 10, et un polymère comprend un nombre de motifs de répétition supérieur à 10.

On comprend dans le présent texte par bio-résorbable (ou résorbable), tout matériau qui, disposé dans un organisme vivant, disparaîtra au terme d'une période déterminée, par exemple à l'issue de trois jours ou encore à l'issue de 3 mois ou 6 mois ou davantage de temps.

L'homme du métier sait quel matériau bio-résorbable choisir, en particulier selon son degré de polymérisation et/ou son degré de réticulation et/ou sa nature chimique, pour obtenir une résorption au terme d'une période déterminée.

Avantageusement, le dispositif implantable est un ligament artificiel ou un tendon artificiel, en particulier ostéo-conducteur et/ou ostéo-inducteur.

Avantageusement, la matrice polymère est hydrosoluble, c'est-à-dire qu'au contact de l'eau, en particulier au contact d'un milieu physiologique, ladite matrice se dissout progressivement, par exemple ladite matrice est dissoute totalement au terme de 3 mois ou 6 mois ou davantage selon les besoins.

En particulier, la matrice polymère est hydrosoluble au contact du milieu aqueux de la zone de l'organisme vivant dans lequel elle est implantée.

En particulier, la matrice polymère est hydrosoluble au contact d'un milieu comprenant de l'eau et ayant une température supérieure ou égale à 20°C, en particulier supérieure ou égale à 25°C ou 30°C ou 35°C ou 37°C. De préférence, le milieu aqueux comprend au moins 50% ou 60% ou 70% ou 80% ou 90% en masse ou en volume d'eau, en particulier d'eau distillée et/ou osmosée.

Le milieu aqueux peut être un milieu physiologique, en particulier un milieu aqueux comprenant de l'eau distillée, et du chlorure de sodium (par exemple dilué à raison de 0,9% en masse de NaCl par rapport au volume du milieu), et éventuellement d'autres minéraux tels que le chlorure de potassium, le chlorure de calcium, le sulfate de magnésium ou encore un mélange de ces derniers.

Avantageusement, un ou plusieurs agent(s) fonctionnel(s), en particulier le ou les agent(s) fonctionnel(s), est/sont dispersé(s) dans la matrice polymère bio-érodable.

De préférence, la matrice polymère bio-érodable comprend le ou les agent(s) fonctionnel(s). De préférence, la masse de la matrice polymère bio-érodable et d'un ou plusieurs agent(s) fonctionnel(s) est supérieure ou égal à 0,05 g/mètre de dispositif implantable et inférieure ou égale à 10 g/mètre de dispositif implantable, encore de préférence inférieure ou égale à 5 g/mètre ou à 3 g/mètre ou à 2 g/mètre ou à 1 g/mètre.

De préférence, le dispositif implantable a une structure globale longiligne et a une longueur déterminée (cm).

### Agent(s) fonctionnel(s)

Avantageusement, on comprend dans le présent texte par agent fonctionnel, tout agent ayant une fonction d'intégration osseuse, en particulier ostéo-conducteur et/ou ostéo-inducteur, et/ou tout agent ayant une fonction prophylactique et/ou une fonction curative d'une pathologie donnée.

On comprend dans le présent texte par agent fonctionnel ostéo-inducteur, tout agent permettant d'induire la différenciation des cellules souches en cellules osseuses, en particulier sans réaction immunitaire.

On comprend dans le présent texte par agent fonctionnel ostéo-conducteur, tout agent fonctionnel favorisant la formation d'os.

### Ame longiligne

On comprend avantageusement dans le présent texte par âme longiligne, tout élément ayant une longueur nettement supérieure à son épaisseur ou à sa largeur, par exemple une longueur supérieure d'au moins 5 ou 8 ou 10 ou 15 ou 20 ou 30 fois la largeur ou l'épaisseur de ladite âme longiligne.

L'âme longiligne est de préférence une âme longiligne textile.

L'âme longiligne peut comprendre (ou peut être) un ou plusieurs fil(s) retordu(s), guipé(s) ou tressé(s), par exemple il s'agit d'une tresse, en particulier pleine; une bande tricotée étroite ou tissée étroite, par exemple il peut s'agir d'un tube tricoté ; ou une bande dans un ou plusieurs non-tissés, notamment thermo-soudés.

De préférence, l'âme longiligne textile comprend un ou des fil(s) et/ou des fibres.

De préférence, l'âme longiligne est non-résorbable.

On comprend dans le présent texte par non-résorbable, tout matériau ou structure qui implanté dans un organisme vivant ne se résorbera pas, c'est-à-dire restera pendant plusieurs années sans dégradation de sa structure significative.

L'âme longiligne textile est de préférence une tresse, notamment pleine (c'est-à-dire non creuse).

L'âme longiligne a de préférence un diamètre inférieur ou égal à 20 mm ou 15 mm ou 10 mm ou 8 mm ou 5 mm.

L'âme longiligne a de préférence un diamètre supérieur ou égal à 1 mm ou 2 mm ou 3 mm. L'âme longiligne a de préférence une longueur supérieure ou égale à 10 mm ou 20 mm ou 30 mm ou 40 mm ou 50 mm ou 80 mm.

L'âme longiligne a de préférence une longueur inférieure ou égale à 150 mm ou 130 mm ou 110 mm ou 90 mm ou 70 mm ou 50 mm ou 40 mm ou 30 mm.

Avantageusement, l'âme longiligne a une surface extérieure orientée directement en regard de la surface intérieure du membre longiligne creux.

Avantageusement, l'âme longiligne a une masse linéique supérieure à 0 g/mètre linéaire et inférieure ou égale à 10 g/mètre linéaire ou 8 g/mètre linéaire ou 5 g/mètre linéaire ou 4 g/mètre linéaire ou 3g/mètre linéaire ou 2 g/mètre linéaire ou 1g/mètre linéaire, par exemple est comprise entre 0,50 g/mètre linéaire et 0,80 g/mètre linéaire. Avantageusement, l'âme longiligne a une surface poreuse, c'est-à-dire comprenant des ouvertures, éventuellement traversantes. Les pores sont avantageusement formés par les interstices entre le ou les fils et/ou les fibres.

Dans un mode de réalisation, l'âme longiligne comprend entre 8 et 20 mèches textiles, notamment tressées, en particulier chaque mèche textile comprend un ou plusieurs fil(s). Dans un mode de réalisation, l'âme longiligne (en particulier la couverture auxiliaire textile) comprend entre 12 et 18 mèches textiles (par exemple de l'ordre de 16 mèches textiles), notamment tressées, en particulier chaque mèche textile comprend un ou plusieurs fil(s).

De préférence, l'âme longiligne textile comprend plusieurs fils multi-filamentaires, encore de préférence ayant chacun un titre supérieur ou égal à 50 dtex et inférieur ou égal à 500 dtex ou à 350 dtex ou à 300 dtex.

De préférence, l'âme longiligne textile, notamment tressée, comprend un premier fil multi-filamentaire ayant un titre T1 (dtex), et un second fil multi-filamentaire ayant un titre T2 (dtex), le ratio T2/T1 étant supérieur ou égal à 1,5 ; notamment supérieur ou égal à 2.

De préférence, l'âme longiligne textile comprend :
- une âme auxiliaire comprenant un ou plusieurs fil(s) (notamment deux ou trois fils), en particulier multi-filamentaires, plus particulièrement dont au moins l'un desdits fils (notamment les trois) a un titre (dtex) allant de 100 dtex ou 150 dtex à 300 dtex ou à 250 dtex ; et
- une couverture auxiliaire textile, notamment tressée, autour de ladite âme auxiliaire, en particulier comprenant un ou plusieurs fil(s) multi-filamentaire(s), plus particulièrement comprend lesdits premier et second fils multi-filamentaires décrits au paragraphe précédent. Avantageusement, l'âme auxiliaire comprend un ou plusieurs fil(s) multi-filamentaire(s) non retordu(s) ou disposé(s) sensiblement parallèlement les uns aux autres.

### Membre de couverture longiligne creux

On comprend avantageusement dans le présent texte par membre de couverture longiligne creux, un membre de couverture ayant une longueur nettement supérieure à son épaisseur ou à sa largeur, par exemple une longueur supérieure d'au moins 5 ou 8 ou 10 ou 15 ou 20 ou 30 fois la largeur ou l'épaisseur dudit membre de couverture.

Le membre longiligne creux est de préférence un membre longiligne creux textile.

De préférence, le membre de couverture longiligne creux et l'âme longiligne sont disposés coaxialement, en particulier leurs axes longitudinaux centraux respectifs sont sensiblement confondus.

De manière générale, on comprend par textile dans le présent texte, tout élément (âme, membre creux,...) obtenu par la manipulation d'un ou plusieurs fil(s) et/ou de fibre(s).

De préférence, le membre de couverture longiligne creux est un membre longiligne textile, et peut comprendre (ou peut être) un tube tricoté ; une tresse creuse ; ou un tube dans un ou plusieurs non-tissés, notamment thermo-soudés.

De préférence, le membre longiligne creux textile comprend un ou des fil(s) et/ou des fibres. De préférence, le membre longiligne creux est non-résorbable.

Le membre longiligne textile est de préférence une tresse creuse.

Le membre longiligne creux a de préférence un diamètre extérieur inférieur ou égal à 20 mm ou 15 mm ou 10 mm ou 8 mm ou 5 mm.

Le membre longiligne creux a de préférence un diamètre extérieur supérieur ou égal à 1 mm ou 2 mm ou 3 mm.

Le membre longiligne creux a de préférence une longueur supérieure ou égale à 10 mm ou 20 mm ou 30 mm ou 40 mm ou 50 mm ou 80 mm.

Le membre longiligne creux a de préférence une longueur inférieure ou égale à 150 mm ou 130 mm ou 110 mm ou 90 mm ou 70 mm ou 50 mm ou 40 mm ou 30 mm. Avantageusement, le membre de couverture longiligne creux a une surface intérieure orientée directement en regard de la surface extérieure de l'âme longiligne. Avantageusement, le membre de couverture longiligne creux a une surface extérieure sensiblement opposée à sa surface intérieure.

De préférence, l'épaisseur de la paroi du membre longiligne creux s'étend entre sa surface intérieure et sa surface extérieure.

Avantageusement, le membre de couverture longiligne creux a une masse linéique supérieure à 0 g/m² et inférieure ou égale à 10 g/mètre linéaire ou à 8 g/mètre linéaire ou à 5 g/mètre linéaire ou à 4 g/mètre linéaire ou à 3 g/mètre linéaire ou à 2 g/mètre linéaire ou à 1g /mètre linéaire, par exemple est comprise entre 0,50 g/mètre linéaire et 0,90 g/mètre linéaire.

Avantageusement, le membre de couverture longiligne creux comprend des orifices traversants, en particulier s'étendant entre sa surface intérieure et sa surface extérieure, notamment débouchant sur lesdites surfaces intérieure et extérieure du membre de couverture longiligne creux.

Les pores sont avantageusement formés par les interstices entre le ou les fil(s) et/ou les fibres. Ces orifices permettent de mettre en communication fluidique le volume intérieur du membre longiligne avec l'extérieur et donc sa surface extérieure. Ainsi, le ou les agent(s) fonctionnel(s) contenu(s) dans la matrice polymère bio-érodable est/sont libéré(s) au fur et à mesure de sa dégradation dans le volume intérieur puis selon la surface extérieure du membre longiligne. La surface extérieure du membre longiligne permet ainsi avantageusement de former une surface extérieure d'ostéo-intégration.

De préférence, le membre longiligne creux est surtricoté ou surtressé, de préférence surtressé, autour de l'âme longiligne.

Dans un mode de réalisation, le membre longiligne comprend entre 8 et 20 mèches textiles, notamment tressées, en particulier chaque mèche textile comprend un ou plusieurs fil(s). Dans un mode de réalisation, le membre longiligne comprend entre 10 et 18 mèches textiles tressées (en particulier 12 ou 16 mèches tressées), en particulier chaque mèche textile comprend un ou plusieurs fil(s), plus particulièrement entre 3 et 8 fils.

De préférence, le membre longiligne creux, notamment tressé, comprend plusieurs fils multi-filamentaires (notamment tressés), encore de préférence entre 30 et 75 ou 70 fils multi-filamentaires, préférentiellement entre 40 et 70 fils multifilamentaire(s), notamment entre 50 et 70 fils multi-filamentaires ou entre 55 et 70 fils multi-filamentaires ou encore entre 60 et 68 fils multifilamentaires, chaque fil ayant un titre supérieur ou égal à 50 dtex ou à 80 dtex ou à 100 dtex et inférieur ou égal à 300 dtex ou à 250 dtex ou 200 dtex ou à 150 dtex.

### Orifices traversants

Les orifices traversants peuvent avoir n'importe quelle forme, ils peuvent être de forme ovoïde ou polygonale notamment.

Lorsque les orifices sont ovoïdes, les orifices sont de préférence circonscrits dans un cercle de diamètre de 20 µm à 5000 µm, encore de préférence de diamètre de 20 µm à 500 µm. Lorsque les orifices sont des polygones, les longueurs des côtés des polygones sont de préférence supérieures ou égales à 20 µm et inférieures ou égales à 5000 µm, encore de préférence supérieures ou égales à 20 µm et inférieures ou égales à 500 µm.

### Fil(s)/Fibre(s)

Le ou les fil(s) (en particulier de l'âme longiligne et/ou du membre de couverture et/ou de l'âme longiligne auxiliaire et/ou du membre de couverture auxiliaire) peut/peuvent être un fil mono-filamentaire, un fil filé de fibres, ou un fil multi-filamentaire.

Le ou les fil(s) et/ou les fibres peut/peuvent comprendre un matériau (ou être dans un matériau) choisi parmi : les polyesters, notamment le polyéthylène téréphtalate (PET), par exemple le Dacron^{®}, et le poly butylène téréphtalate (PBT) ; les polyamides, tels que le PA 6-6, le PA 6, le PA 4-6 ; les polyoléfines, tels que le polypropylène, le polyéthylène (notamment de basse densité ou de haute ou très haute densité) ; les polymères d'acide lactique et éventuellement d'acide glycolique, tels que le PLLA, le PLDA, et le PLGA.

Le ou les fil(s) et/ou les fibres peut/peuvent être biorésorbable(s), non-biorésorbable(s), ou semi-biorésorbable(s), de préférence non-biorésorbable(s).

Avantageusement, un fil multifilamentaire comprend un nombre de filaments compris entre 5 et 30, en particulier entre 5 et 20 filaments.

Avantageusement, un filament d'un fil multifilamentaire a une masse linéique comprise entre 1 dtex et 30 dtex, de préférence entre 5 dtex et 20 dtex.

De préférence, le ou les fil(s) comprend/comprennent au moins un matériau choisi parmi : les polyester(s) ; notamment le polyéthylène téréphtalate (PET), par exemple le Dacron^{®}, ou le poly butylène téréphtalate (PBT) ; ou les polyoléfines, tels que le polypropylène, le polyéthylène (notamment de basse densité ou de haute ou très haute densité) ; ou encore un mélange de ces derniers.

De préférence, le ou les fil(s) sont en polyéthylène téréphtalate (PET), par exemple le Dacron^{®}, ou en poly butylène téréphtalate (PBT) ; ou en polypropylène ou en polyéthylène; ou encore un mélange de ces derniers (par exemple un ou des fil(s) est/sont en PET ou en PBT et un ou des fil(s) est/sont en PP ou en PE).

Avantageusement, la matrice polymère bio-érodable forme un revêtement polymère revêtant au moins en partie la surface extérieure de l'âme longiligne, en particulier la surface extérieure de la couverture auxiliaire textile.

Avantageusement, la matrice polymère bio-érodable imprègne à cœur l'âme longiligne.

En particulier, on comprend par imprègne à cœur que l'âme longiligne comprend de la matrice polymère revêtant au moins en partie l'âme auxiliaire textile et la couverture auxiliaire textile.

Dans une variante, l'âme longiligne textile, notamment comprenant une âme auxiliaire textile et une couverture auxiliaire textile disposé autour au moins en partie de ladite âme auxiliaire textile, et le membre de couverture creux textile forment un ensemble textile ayant un titre total supérieur ou égal à 18 000 dtex et inférieur ou égal à 25 000 dtex, de préférence inférieur ou égal à 24 000 dtex, encore de préférence inférieur ou égal à 22 000 dtex, en particulier supérieur ou égal à 20 000 dtex, plus particulièrement supérieur ou égal à 21 000 dtex.

Dans une variante, le ou les agent(s) fonctionnel(s) comprend/comprennent un ou plusieurs agent(s) ostéo-inducteur(s) et/ou ostéo-conducteur(s).

Dans une variante, le ou les agent(s) ostéo-inducteur(s) et/ou ostéo-conducteur(s) est/sont choisi(s) parmi : les bioverres ; les biocéramiques à base de phosphate de calcium, notamment les hydroxyapatites, et le phosphate tricalcique, et un mélange de ces derniers. Avantageusement, le ou les agent(s) ostéo-inducteur(s) et/ou ostéo-conducteur(s), ou le ou les agent(s) ostéo-inducteur(s) et/ou ostéo-conducteur(s) comprend/comprennent un ou des agent(s) ostéo-inducteur(s) et/ou ostéo-conducteur(s), qui est/sont choisi(s) parmi les bioverres.

Avantageusement, le ou les agent(s) ostéo-inducteur(s) et/ou ostéo-conducteur(s), ou le ou les agent(s) ostéo-inducteur(s) et/ou ostéo-conducteur(s) comprend/comprennent un ou des agent(s) ostéo-inducteur(s) et/ou ostéo-conducteur(s), qui est/sont choisi(s) parmi des biocéramiques à base de phosphate de calcium, notamment les hydroxyapatites, et le phosphate tricalcique, ou encore un mélange de ces derniers.

De préférence, l'hydroxyapatite est un phosphate de calcium qui appartient à la famille des apatites, il s'agit d'un nom générique désignant des phosphates de composition variable.

De préférence, le bioverre est le bioverre 45S5. Avantageusement, ce bioverre est ostéo-conducteur et ostéo-inducteur.

De préférence, les bioverres sont des biocéramiques comprenant de la silice, du sodium, du calcium et du phosphate, en particulier selon des quantités variables, plus particulièrement la fraction massique en silice (notamment en SiO₂) est majoritaire par rapport aux autres composants.

Le ou les agent(s) ostéo-inducteur(s) et/ou ostéo-conducteur(s) est/sont hydrosoluble(s) ou non hydrosoluble(s), de préférence hydrosoluble(s).

De préférence, le ou les agent(s) ostéo-inducteur(s) et/ou ostéo-conducteur(s) est/sont poreux.

De préférence, le bioverre comprend :
- de 30% en masse à 55% en masse de SiO₂, en particulier de 40% en masse à 50% en masse de SiO₂ ; et/ou
- de 15% en masse à 35% en masse de Na₂O, en particulier de 20% en masse à 30% en masse de Na₂O ; et/ou
- de 15% en masse à 35% en masse de CaO, en particulier de 20% en masse à 30% en masse de CaO ; et/ou
- de 2% en masse à 15% en masse de P₂O₅, en particulier de 4% en masse à 10% en masse de P₂O₅.

Dans une variante, le ou les agent(s) fonctionnel(s) est/sont choisi(s) parmi : les anti-inflammatoires, les antibiotiques, les antibactériens, les antalgiques, et un mélange de ces derniers.

Dans une variante, la matrice polymère bio-érodable comprend un ou plusieurs agent(s) fonctionnel(s) qui est/sont un ou des agent(s) ostéoinducteur(s) et/ou ostéoconducteur(s), et éventuellement un ou plusieurs autres agent(s) fonctionnel(s) choisi(s) parmi : les anti-inflammatoires, les antibiotiques, les antibactériens, les antalgiques, et un mélange de ces derniers.

Dans une variante, l'âme longiligne comprend une âme longiligne textile, en particulier une tresse.

Dans une variante, l'âme longiligne textile est revêtue au moins en partie de la matrice polymère bio-érodable dans laquelle le ou les agent(s) fonctionnel(s) est/sont dispersé(s).

Le revêtement dans la matrice polymère bio-érodable peut être continu, par exemple sous forme de film, ou discontinu, par exemple disposé sous forme de points, de bandes, de motifs variés ou une combinaison de ces derniers.

Avantageusement, l'âme longiligne est revêtue selon sa surface extérieure de la matrice bio-érodable comprenant un ou plusieurs agent(s) fonctionnel(s), totalement ou en partie.

Dans une variante, le membre de couverture longiligne creux comprend un membre de couverture longiligne textile, en particulier comprend une tresse creuse.

Avantageusement, le membre de couverture est surtressé autour de l'âme longiligne de sorte que l'ensemble est parfaitement homogène et a une bonne cohésion.

Dans une variante, la matrice polymère bio-érodable comprend un polymère, ou plusieurs polymères mélangé(s), déshydraté(s) et apte(s) à former un gel soluble en milieu aqueux. Avantageusement, le ou lesdits polymère(s) déshydraté(s) est/sont un ou des polymère(s) bio-érodable(s), en particulier bio-résorbable(s).

Avantageusement, la matrice polymère est solide à la température ambiante, c'est-à-dire à une température supérieure ou égale à 0°C et inférieure ou égale à 40°C ou 37°C ou 30°C, la matrice polymère comprenant au plus 10% ou 5% en masse d'eau par rapport à sa masse totale (comprenant le ou les agent(s) fonctionnel(s)).

Lorsque la matrice polymère est en contact avec un milieu aqueux, la matrice absorbe l'eau, peut éventuellement gonfler selon le polymère utilisé, puis se dissout progressivement en libérant le ou les agent(s) fonctionnel(s) dispersé(s) qu'elle renferme.

On comprend dans le présent texte par un polymère déshydraté tout polymère apte à former un gel en milieu aqueux puis à se solubiliser au fur et à mesure. Le polymère n'est pas sous la forme d'un gel avant implantation. Il est donc déshydraté.

Dans une variante, la matrice polymère bio-érodable comprend un ou plusieurs polymère(s) bio-résorbable(s) choisi(s) parmi les polymères suivants : des (co)polymères de cyclodextrine(s) et/ou de dérivé(s) de cyclodextrine(s) et/ou de complexe(s) d'inclusion de cyclodextrine(s) et/ou de dérivé(s) de cyclodextrine(s) solubles ; un dérivé de polysaccharide réticulé ou non tels que les polymères de collagène ; les polymères dérivés de l'acide hyaluronique ; les polymères de carboxyméthylcellulose ou dérivés de la carboxyméthylcellulose ; les polymères vinyliques tels que les polymères de polyvinylpyrrolidone (PVP) ou les polymères de polyvinylpolypyrrolidone (crospovidone) ; les polymères de polyéthylène glycol ; les polymères d'acide lactique ; les copolymères d'acide lactique, et de polyéthylèneglycol (PLLA/PEG) ; les copolymères d'acide lactique, d'acide glycolique et de polyéthylèneglycol (PLGA/PEG) ; les polymères d'alcools polyvinylique (PVA) ; et les polymères de polyacrylates tel que le polyhydroxyéthylméthacrylate (PHEM) ; et un mélange de ces derniers.

Dans un mode de réalisation, la matrice polymère bio-érodable comprend un ou plusieurs polymère(s) bio-résorbable(s) choisi(s) parmi les polymères vinyliques tels que les polymères de polyvinylpyrrolidone (PVP) ou les polymères de polyvinylpolypyrrolidone (crospovidone), ou un mélange de ces derniers.

Dans un mode réalisation, la matrice polymère bio-érodable comprend un ou plusieurs polymère(s) bio-résorbable(s) choisi(s) parmi: des (co)polymères de cyclodextrine(s) et/ou de dérivé(s) de cyclodextrine(s) et/ou de complexe(s) d'inclusion de cyclodextrine(s) et/ou de dérivé(s) de cyclodextrine(s) solubles, ou un mélange de ces derniers.

Dans un mode réalisation, la matrice polymère bio-érodable comprend un ou plusieurs polymère(s) bio-résorbable(s) choisi(s) parmi :
- un dérivé de polysaccharide réticulé ou non tels que les polymères de collagène ; ou
- les polymères dérivés de l'acide hyaluronique ; ou
- les polymères de carboxyméthylcellulose ou dérivés de la carboxyméthylcellulose ; ou
- un mélange de ces derniers.

Le ou les polymère(s) bio-résorbable(s) de la matrice polymère bio-érodable est/sont de préférence hydrolosuble(s).

Les (co)polymères de cyclodextrine(s) et/ou de dérivé(s) de cyclodextrine(s) et/ou de complexe(s) d'inclusion de cyclodextrine(s) sont de préférence hydrosolubles.

Ces (co)polymères peuvent être obtenus en 1/ préparant à l'état solide un mélange de cyclodextrine(s) et/ou de dérivé(s) de cyclodextrine(s) et/ou de complexe(s) d'inclusion de cyclodextrine(s) et/ou de dérivé(s) de cyclodextrine(s), et d'un acide poly(carboxylique) ou d'un anhydride d'acide poly(carboxylique) ou d'un mélange d'acides poly(carboxyliques) et/ou d'anhydrides d'acides poly(carboxyliques) et, éventuellement, d'un catalyseur ; puis en 2/ chauffant ledit mélange solide, à une température comprise entre 100°C et 200°C, pendant une durée comprise entre 1 min et 60 min, de préférence égale à 30 min.

De préférence, pour préparer le mélange à l'état solide, on prépare une solution aqueuse de cyclodextrine(s) et/ou de dérivé(s) de cyclodextrine(s) et/ou de complexe(s) d'inclusion de cyclodextrine et/ou de dérivé(s) de cyclodextrine et d'un acide poly(carboxylique) et/ou d'un anhydride d'acide polycarboxylique et/ou d'un mélange d'acides poly(carboxyliques) et/ou d'anhydrides d'acides poly(carboxyliques) et, éventuellement, d'un catalyseur et on évapore l'eau de ladite solution à une température comprise entre 40°C et 100°C, de préférence sous vide à 90°C.

De préférence, le catalyseur est choisi parmi les dihydrogénophosphates, les hydrogénophosphates, les phosphates, les hypophosphites, les phosphites de métaux alcalins, les sels de métaux alcalins des acides polyphosphoriques, les carbonates, les bicarbonates, les acétates, les borates, les hydroxydes de métaux alcalins, les amines aliphatiques et l'ammoniaque, et de préférence, parmi l'hydrogénophosphate de sodium, le dihydrogénophosphate de sodium et l'hypophosphite de sodium.

De préférence, la cyclodextrine est choisie parmi l'α-cyclodextrine, la β-cyclodextrine et la γ-cyclodextrine et les dérivés de cyclodextrine sont choisis parmi les dérivés hydroxypropyl méthylés ou acétylés de l'α-cyclodextrine, de la β-cyclodextrine et de la γ-cyclodextrine et les complexes d'inclusion desdites cyclodextrines et desdits dérivés de cyclodextrine(s).

De préférence, l'acide poly(carboxylique) et l'anhydride d'acide polycarboxylique sont choisis parmi les acides polycarboxyliques et les anhydrides des acides poly(carboxyliques) suivants: les acides poly(carboxyliques) acycliques saturés et insaturés, les acides poly(carboxyliques) cycliques saturés et insaturés, les acides poly(carboxyliques) aromatiques, les acides hydroxypoly(carboxyliques), de préférence, parmi l'acide citrique, l'acide poly(acrylique), l'acide poly(méthacrylique), l'acide 1, 2, 3, 4-butane tétracarboxylique, l'acide maléique, l'acide citraconique, l'acide itaconique, l'acide 1, 2, 3-propane tricarboxylique, l'acide aconitique, l'acide all-cis-1, 2, 3, 4-cyclopentanetétracarboxylique, l'acide méllitique, l'acide oxydisuccinique, l'acide thiodisuccinique.

De préférence, l'acide poly(carboxylique) est choisi parmi les acides poly(carboxyliques) acycliques saturés ou insaturés, les acides poly(carboxyliques) cycliques saturés et insaturés, les acides poly(carboxyliques) aromatiques, les acides hydroxypoly(carboxyliques), de préférence, parmi l'acide citrique, l'acide poly(acrylique), l'acide poly(méthacrylique), l'acide 1, 2, 3, 4-butanetétracarboxylique, l'acide 1, 2, 3-propane tricarboxylique, l'acide aconitique, l'acide all-cis-1, 2, 3, 4-cyclopentanetétracarboxylique, l'acide méllitique, l'acide oxydisuccinique, l'acide thiodisuccinique.

Dans une variante, la matrice polymère bio-érodable comprend un ou plusieurs polymère(s) choisi(s) parmi : les polymères de polyvinylpyrrolidone (PVP) ; les polymères d'alcool polyvinylique ; les (co)polymères de cyclodextrine(s) et/ou de dérivé(s) de cyclodextrine(s) et/ou de complexe(s) d'inclusion de cyclodextrine(s), en particulier hydrosolubles, ou un mélange de ces derniers.

Dans une variante, la matrice polymère bio-érodable comprend un ou plusieurs polymère(s) bio-érodable(s), et le ratio massique (agent(s) fonctionnel(s) / polymère(s) bio-érodable(s)) dans la matrice polymère, en particulier le ratio massique (agent(s) ostéo-inducteur(s) et/ou ostéo-inducteur(s) / polymère(s) bio-érodable(s)) dans la matrice polymère, est supérieur ou égal à 0,20 et inférieur ou égal à 1,8 ; de préférence supérieur ou égal à 0,20 et inférieur ou égal à 1 ; encore de préférence supérieur ou égal à 0,40 et inférieur ou égal à 0,80 ; préférentiellement supérieur ou égal à 0,50 et inférieur ou égal à 0,70.

Dans une variante, le membre de couverture longiligne creux comprend une tresse comprenant de 8, en particulier de 12, à 30 mèches tressées, et chaque mèche tressée comprend de 2 à 8 fils, de préférence lesdits fils ont chacun un titrage s'étendant de 80 dtex à 200 dtex.

De préférence, chaque mèche tressée comprend de 3 à 8 fils, en particulier de 3 à 7 fils, plus particulièrement de 3 à 6 fils, éventuellement de 5 à 7 fils, par exemple 4 fils.

De préférence, le membre de couverture comprend de 20 à 28 mèches tressées.

De préférence, le membre de couverture comprend de 8 à 18 mèches tressées, en particulier de 10 à 18 mèches tressées, plus particulièrement de 12 à 16 mèches tressées, par exemple 12 mèches tressées ou 16 mèches tressées.

Cette structure particulière permet d'obtenir des orifices traversants dont les dimensions sont optimisées pour la migration progressive du ou des agent(s) fonctionnel(s) selon la surface extérieure du membre de couverture, et notamment la formation d'une surface extérieure d'ostéo-intégration lorsqu'il y a un ou des agent(s) ostéo-inducteur(s) et/ou ostéo-conducteur(s).

Dans une variante, le membre de couverture longiligne creux comprend entre 30 et 120 fils, de préférence entre 30 fils et 80 fils, notamment lesdits fils ne sont pas biorésorbables.

De préférence, le membre de couverture longiligne creux tressé comprend de 40 fils tressés à 75 fils tressés, encore de préférence de 45 fils tressés à 68 fils tressés, par exemple de 60 fils tressés à 68 fils tressés.

De préférence, au moins l'un desdits fils est un fil multifilamentaire, en particulier lesdits fils sont des fils multifilamentaires.

Le membre de couverture offre de bonnes performances mécaniques tout en offrant une déformabilité du fait de nombre important de fils multi-filamentaires.

Dans une variante, la fraction massique de la matrice polymère bio-érodable comprenant le ou les agent(s) fonctionnel(s) dans ledit dispositif implantable est supérieure ou égale à 5% et inférieure ou égale à 25%, de préférence supérieure ou égale à 10% et inférieure ou égale à 20%.

Avantageusement, la fraction massique de la matrice polymère bio-érodable et en agent(s) fonctionnel(s) est mesurée par rapport à la masse totale du dispositif implantable, en particulier comprenant la matrice polymère, le ou les agent(s) fonctionnel(s), l'âme longiligne et le membre de couverture longiligne creux.

Dans une variante, la fraction massique de l'âme longiligne (en particulier de l'âme longiligne nue), exempte de la matrice polymère et en agent(s) fonctionnel(s), et du membre de couverture longiligne creux (en particulier du membre de couverture longiligne creux nu) mesurée par rapport à la masse totale du dispositif implantable est supérieure ou égale à 50% ou à 60% ou à 70%, de préférence supérieure ou égale à 75%.

La présente invention a pour objet, selon un second aspect, un procédé de fabrication d'un dispositif implantable, notamment selon l'une quelconque des variantes de réalisation en référence au premier aspect de l'invention, comprenant avantageusement :
a) une étape d'application d'un revêtement polymérique bio-érodable comprenant un ou plusieurs agent(s) fonctionnel(s) sur une âme longiligne, notamment textile,
b) une étape de disposition, notamment de surtressage, d'un membre de couverture longiligne creux, notamment textile, autour au moins en partie de ladite âme longiligne, notamment textile, revêtue au moins en partie ;
c) une étape de récupération d'un dispositif implantable comprenant une âme longiligne et un membre de couverture longiligne creux comprenant un volume intérieur recevant au moins en partie ladite âme longiligne, ladite âme longiligne comprend une matrice polymère bio-érodable en milieu aqueux, et un ou plusieurs agent(s) fonctionnel(s), et le membre de couverture comprend des orifices traversants en communication fluidique avec ledit volume intérieur.

Dans un mode de réalisation, l'étape d'application a) comprend une étape d'application (a1) d'une préparation liquide comprenant un ou plusieurs (co)polymère(s) bio-érodable(s), ou un ou plusieurs monomère(s) et/ou oligomère(s) précurseur(s) du ou desdits (co)polymère(s) bio-érodable(s), en milieu aqueux, et comprenant en outre un ou plusieurs agent(s) fonctionnel(s), sur une âme longiligne, notamment textile, et une étape de séchage (a2), suivie éventuellement d'une étape de polymérisation (a3).

L'étape d'application a) peut comprendre une étape d'imprégnation (a1) ou une étape de pulvérisation (a1) ou une étape de trempage (a1), au moyen d'une solution/dispersion comprenant le ou les (co)polymère(s) et/ou le ou les monomère(s) et/ou le ou les oligomère(s) précurseur(s) du ou desdits polymère(s), et éventuellement une étape d'exprimage (a11), par exemple une étape de calandrage (a11). L'étape (a1) et/ou l'étape (a11) peut/peuvent être effectuée(s) plusieurs fois, par exemple au moins 4 fois ou au moins 8 fois selon le taux d'emport désiré.

La préparation liquide peut être une solution/dispersion aqueuse, ou une solution/dispersion solvantée (différente de l'eau) ou encore une solution/dispersion à base d'au moins un alcool, par exemple à base d'éthanol.

De préférence, la fraction massique en (co)polymère(s) bio-érodable(s), ou en monomère(s) ou oligomère(s) précurseur(s) desdits (co)polymère(s) bio-érodable(s), dans la préparation liquide (masse/volume) est supérieure ou égale à 5% et inférieure ou égale à 30%, en particulier supérieure ou égale à 10% et inférieure ou égale à 25%.

L'étape de séchage (a2) comprend de préférence le chauffage de l'âme longiligne revêtue au moins partiellement de la préparation liquide en sorte d'évaporer l'eau et/ou un ou plusieurs solvant(s), par exemple la température de chauffage est comprise entre 70°C et 100°C ou 90°C ou 80°C, pendant 30 minutes ou davantage jusqu'à formation d'une matrice polymère bio-érodable solide.

L'âme longiligne partiellement revêtue peut être chauffée ensuite dans une étape de polymérisation (a3) au-cours de laquelle l'âme longiligne partiellement revêtue est chauffée en sorte de polymériser, éventuellement réticuler, le ou les monomère(s) ou oligomère(s) précurseur(s) desdits (co)polymère(s) bio-érodable(s), par exemple à une température de 150°C pendant au moins 30 minutes s'agissant des (co)polymères de cyclodextrines.

Dans une variante, l'étape b) est une étape de tressage d'une tresse creuse sur un dispositif de tressage (par exemple une tresseuse) comprenant un nombre total de fuseaux supérieur ou égal à 12, plus particulièrement supérieur ou égal à 18, et inférieur ou égal à 30, en particulier inférieur ou égal à 26, et le nombre de fuseaux vides est supérieur ou égal à 10% et inférieur ou égal à 60% du nombre total de fuseaux, en particulier supérieur ou égal à 20% et inférieur ou égal à 60% du nombre total de fuseaux, en particulier le nombre de fuseaux pleins est supérieur ou égal à 40% et inférieur ou égal à 90% du nombre total de fuseaux du dispositif de tressage.

Avantageusement, le nombre de fuseaux pleins est supérieur ou égal à 40%, en particulier supérieur ou égal à 45%, et inférieur ou égal à 90%, en particulier inférieur ou égal à 80%, plus particulièrement inférieur ou égal à 70%, du nombre total de fuseaux du métier à tresser.

Dans un mode de réalisation, le nombre de fuseaux du métier à tresser de l'étape b) de surtressage du membre de couverture est compris entre 20 et 26, en particulier de l'ordre de 24 fuseaux.

Dans un mode de réalisation, le nombre de fuseaux pleins est compris entre 60% et 75% du nombre total de fuseaux du métier à tresser lors de l'étape b).

Dans un mode de réalisation, le nombre de fuseaux pleins est compris entre 10 et 18, en particulier entre 12 et 18, plus particulièrement entre 14 et 18.

On comprend dans le présent texte par fuseau plein d'un métier à tresser que ce fuseau supporte un ou plusieurs fil(s) lors du tressage, et par fuseau vide d'un métier à tresser que ce fuseau ne supporte pas de fil lors du tressage.

Cette disposition des fuseaux pleins et vides sur le métier à tresser permet d'écarter les mèches tressées, et de former des orifices traversants favorisant la migration graduelle et la fixation d'agent(s) fonctionnel(s) sur la surface extérieure du membre de couverture longiligne creux.

De préférence, le nombre de fuseaux (pleins et/ou vides) est un nombre entier.

Dans une variante, l'étape b) est une étape de tressage d'une tresse creuse sur un dispositif de tressage comprenant des fuseaux pleins et des fuseaux vides, et les fuseaux pleins sont répartis dans au moins trois groupes de fuseaux pleins (notamment dans quatre groupes de fuseaux pleins), dont chaque groupe comprend plusieurs fuseaux pleins voisins (notamment deux ou trois ou quatre fuseaux pleins voisins), et un groupe de fuseaux pleins est séparé d'un autre groupe de fuseaux pleins par au moins un fuseau vide, de préférence par au moins deux ou trois fuseaux vides.

Avantageusement, un groupe de fuseaux pleins comprend au moins trois ou quatre fuseaux pleins voisins.

De préférence, le dispositif de tressage comprend de 10 à 30 fuseaux, encore de préférence de 14 à 28 fuseaux, en particulier de 20 à 28 fuseaux, notamment de 22 à 26 fuseaux.

Dans un mode de réalisation, le dispositif de tressage comprend 4 groupes de trois ou quatre fuseaux pleins voisins ou adjacents, et 4 groupes de 3 ou 4 fuseaux vides voisins ou adjacents, un groupe de fuseaux pleins voisins ou adjacents étant disposé entre deux groupes de fuseaux vides.

Dans une variante, le dispositif de tressage comprend trois ou quatre groupes de trois ou quatre fuseaux pleins voisins et trois ou quatre groupes de fuseaux vides voisins, un groupe de fuseaux pleins voisins étant disposé entre deux groupes de fuseaux vides voisins.

Dans une variante, l'étape b) est une étape de tressage d'une tresse creuse avec un angle de tressage sensiblement constant supérieur ou égal à 2 mm et inférieur ou égal à 15 mm, de préférence supérieur ou égal à 4 mm et inférieur ou égal à 10 mm.

Avantageusement, l'angle de tressage peut être mesuré au microscope.

La présente invention a également pour objet, selon un troisième aspect, un dispositif implantable susceptible d'être obtenu par le procédé de fabrication selon un second aspect de l'invention.

En particulier, les variantes/modes de réalisation/définitions selon un premier aspect de l'invention s'appliquent indépendamment les uns des autres au dispositif implantable selon un troisième aspect et au procédé de fabrication selon un second aspect de l'invention.

### Description des dessins

La présente invention sera mieux comprise à la lecture des modes de réalisation suivants, cités à titre non limitatif, et illustrés par les figures dans lesquelles :
[Fig.1] la figure 1 représente schématiquement un premier exemple d'agencement des fuseaux pleins avec des fuseaux vides d'un métier à tresser comprenant 16 fuseaux au total pour le tressage d'un premier exemple de membre de couverture longiligne creux selon l'invention ;
[Fig.2] la figure 2 représente schématiquement un second exemple d'agencement des fuseaux pleins avec des fuseaux vides d'un métier à tresser comprenant 16 fuseaux au total pour le tressage d'un second exemple de membre de couverture longiligne creux selon l'invention ;
[Fig.3] la figure 3 représente schématiquement un troisième exemple d'agencement des fuseaux pleins avec des fuseaux vides d'un métier à tresser comprenant 24 fuseaux au total pour le tressage d'un troisième exemple de membre de couverture longiligne creux selon l'invention ;
[Fig.4] la figure 4 représente schématiquement un quatrième exemple d'agencement des fuseaux pleins avec des fuseaux vides d'un métier à tresser comprenant 24 fuseaux au total pour le tressage d'un quatrième exemple de membre de couverture longiligne creux selon l'invention ;
[Fig.5] la figure 5 est une photographique obtenue par microscopie à balayage électronique de la surface extérieure d'un premier exemple d'âme longiligne selon l'invention revêtue au moins partiellement d'une matrice bio-érodable comprenant au moins un agent fonctionnel ostéo-inducteur et/ou ostéo-conducteur après 7 jours d'immersion dans un fluide corporel de simulation pour étudier la bio-minéralisation ;
[Fig.6] la figure 6 est une photographique obtenue par microscopie à balayage électronique de la surface extérieure du troisième exemple de membre de couverture selon l'invention après 7 jours d'immersion dans un fluide corporel de simulation pour étudier la bio-minéralisation ;

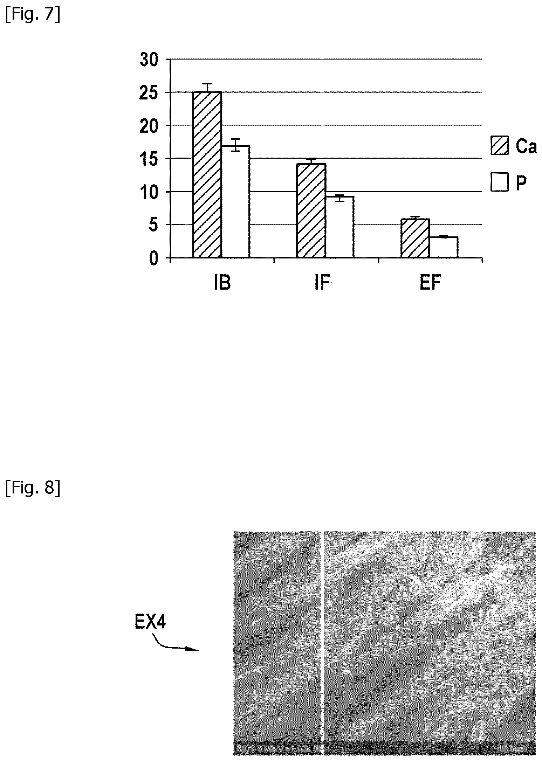

[Fig.7] la figure 7 est un graphique représentant la proportion massique en calcium (Ca) et en phosphore (P) en ordonnées mesurées par spectroscopie de rayons X à dispersion d'énergie sur la surface extérieure d'un exemple d'âme longiligne selon l'invention, référencée (IB) en abscisse, selon la surface intérieure du troisième exemple de membre de couverture selon l'invention, référencée (IF) en abscisse, et selon la surface extérieure du troisième exemple de couverture selon l'invention, référencée (EF), et ce après 7 jours d'immersion dans un fluide corporel de simulation pour étudier la bio-minéralisation ;
[Fig.8] la figure 8 est une photographique obtenue par microscopie à balayage électronique de la surface extérieure du quatrième exemple de membre de couverture selon l'invention après 7 jours d'immersion dans un fluide corporel de simulation pour étudier la bio-minéralisation.

### Description des modes de réalisation

### A- Préparation d'une solution polymérique

Environ 4,82g de poly(vinyl pyrrolidone) (PVP) (de la marque Kollidone^{®} de grade médicale, disponible en poudre, commercialisée par BASF, avec Mw de 900 000 à 1 200 000 g/mol) est dissoute dans 60 ml d'éthanol concentré à 96% (dans de l'eau osmosée, soit 96% masse/volume) à température ambiante graduellement pour éviter une précipitation du polymère, le mélange est agité doucement à 150 rpm pendant au moins 30 minutes.

Puis, environ 3g de bioverre 45S5 sont ajoutés à la solution comprenant la PVP et dispersés dans cette dernière. La concentration en bioverre est ici de 10% en masse/volume de solution.

Les solutions sont laissées sous agitation pendant 8h.

### B- Imprégnation d'une âme longiligne textile

Avantageusement, l'âme longiligne textile comprend une âme auxiliaire comprenant 3 fils multi-filamentaires en polyester (en particulier en polyéthylène téréphtalate) chacun de 190 dtex, et une couverture auxiliaire surtressée autour de ladite âme auxiliaire, ladite couverture auxiliaire comprenant 16 mèches tressées (soit tricotée sur un métier à 16 fuseaux tous pleins), chaque mèche tressée comprend un fil multi-filamentaire en polyester (en particulier en polyéthylène téréphtalate) de 90 dtex et un fil multi-filamentaire en polyester (en particulier en polyéthylène téréphtalate) de 230 dtex. Avantageusement, l'âme longiligne textile a une masse linéique comprise entre 0,50 g/mètre et 1 g/mètre, par exemple de l'ordre de 0,60 g/mètre.

L'âme longiligne est passée dans la solution puis à travers un dispositif d'exprimage, dans cet exemple précis entre deux rouleaux tournant à environ 2 mètre/minute et appliquant une pression d'exprimage de 3 bars. Le nombre de passes d'imprégnation en plein bain et d'exprimage est d'environ 10.

L'âme longiligne est séchée à l'air libre pendant 24h. On obtient ainsi un premier exemple d'âme longiligne.

### C- Surtressage d'un membre de couverture longiligne

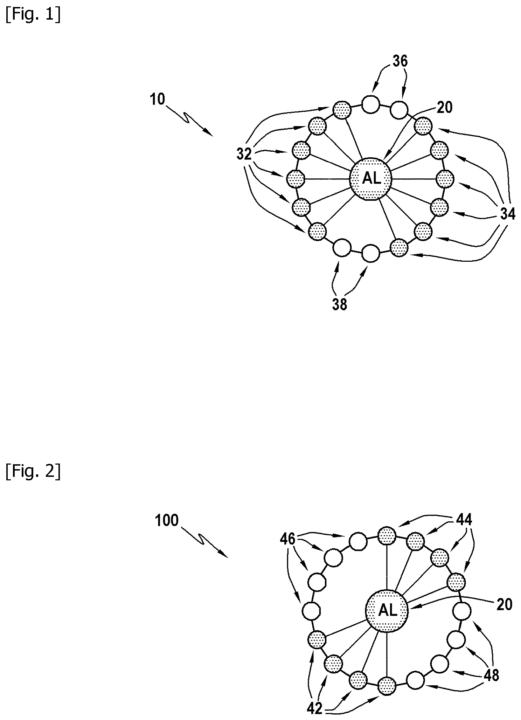

De manière générale dans les exemples 1 à 4 ci-dessous, un fuseau vide est représenté sur les figures 1 à 4 par un rond blanc, et un fuseau plein est représenté par un fuseau rempli avec des points.

### Exemple 1 de dispositif implantable

Un premier exemple de membre de couverture est surtressé, selon le premier exemple d'agencement 10 représenté à la figure 1 autour de l'âme longiligne 20 revêtue obtenue au point B. La figure 1 représente la disposition des fuseaux d'un métier à tresser de 16 fuseaux. Dans cet exemple précis, un premier groupe de 6 fuseaux pleins voisins 32 (ou adjacents) est espacé d'un second groupe de 6 fuseaux pleins voisins (ou adjacents) par des premier et second groupes de chacun deux fuseaux vides 36 et 38. Chaque fuseau plein supporte 6 fils multi-filamentaires en polyéthylène téréphtalate de 138 dtex chacun. On obtient ainsi le premier exemple de dispositif implantable selon l'invention (EX1).

### Exemple 2 de dispositif implantable

Un second exemple de membre de couverture est surtressé, selon le deuxième exemple d'agencement 100 représenté à la figure 2 autour de l'âme longiligne 20 revêtue obtenue au point B. La figure 2 représente la disposition des fuseaux d'un métier à tresser de 16 fuseaux. Dans cet exemple précis, un premier groupe de 4 fuseaux pleins voisins (ou adjacents) 42 est espacé d'un second groupe de 4 fuseaux pleins voisins (ou adjacents) 44 par des premier et second groupes de chacun 4 fuseaux vides 46 et 48. Chaque fuseau plein supporte 6 fils multi-filamentaires en polyéthylène téréphtalate de 138 dtex chacun. On obtient ainsi le second exemple de dispositif implantable selon l'invention (EX2).

### Exemple 3 de dispositif implantable

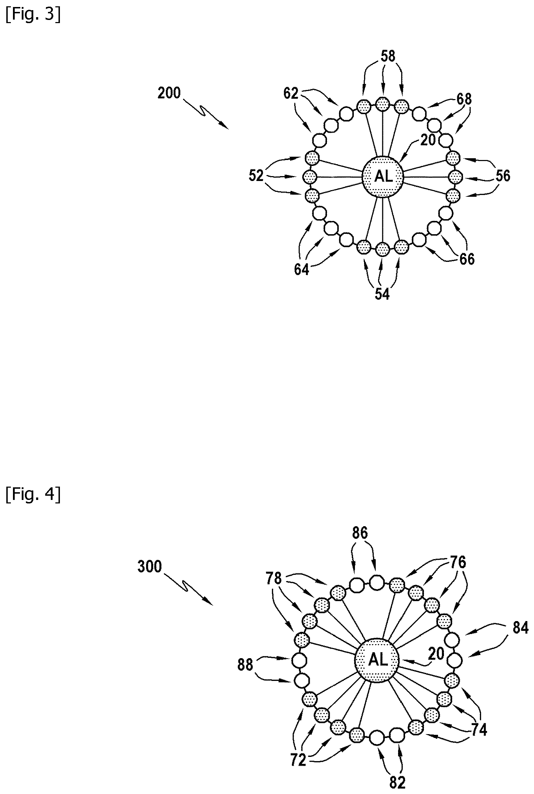

Un troisième exemple de membre de couverture est surtressé, selon le troisième exemple d'agencement 200 représenté à la figure 3 autour de l'âme longiligne 20 revêtue obtenue au point B. La figure 3 représente la disposition des fuseaux d'un métier à tresser de 24 fuseaux. Dans cet exemple précis, quatre groupes de chacun 3 fuseaux pleins voisins (adjacents) 52,54, 56 et 58 sont espacés entre-eux à chaque fois par un groupe de 3 fuseaux vides 62,64,66 et 68. Ainsi, un groupe de 3 fuseaux pleins est entouré de deux groupes de chacun 3 fuseaux vides. Chaque fuseau plein supporte 4 fils multi-filamentaires en polyéthylène téréphtalate de 138 dtex chacun. On obtient ainsi le troisième exemple de dispositif implantable selon l'invention (EX3).

### Exemple 4 de dispositif implantable

Un quatrième exemple de membre de couverture est surtressé, selon le quatrième exemple d'agencement 300 représenté à la figure 4 autour de l'âme longiligne revêtue obtenue au point B. La figure 4 représente la disposition des fuseaux d'un métier à tresser de 24 fuseaux. Dans cet exemple précis, quatre groupes de chacun 4 fuseaux pleins voisins (ou adjacents) 72,74,76 et 78 sont espacés entre-eux à chaque fois par un groupe de 2 fuseaux vides 82,84,86 et 88. Ainsi, un groupe de 4 fuseaux pleins 72,74,76 ou 78 est entouré de deux groupes de 2 fuseaux vides (82,84,86,88). Chaque fuseau plein supporte 4 fils multi-filamentaires en polyéthylène téréphtalate de 138 dtex chacun. On obtient ainsi le quatrième exemple de dispositif implantable selon l'invention (EX4).

### D- Etude la bio-minéralisation

7500 ml d'un fluide corporel de simulation (SBF) est préparé selon le protocole de Tadashi Kokubo et Takadama, Biomaterials, 1er mai 2006, 27(15) : 2907-15. Les réactifs sont introduits dans de l'eau ultrapure dans .cet ordre : NaCl (60,2625g), NaHCO₃ (2,6625g), KCI (1,6875g), K₂HPO₄, 3H₂O (1,7325g), MgCl₂, 6H₂O (2,3325g), 1Mol/L de HCl, CaCl₂ (2,19g), Na₂SO₄ (0,54g), 293 ml de HCl à 1Mol/l est introduit pour ajuster le pH à 7.40.

Au moins 3 échantillons de 3 cm de longueur chacun pour les exemples EX1, EX2, EX3 et EX4 sont immergés dans 120ml de la solution de SBF selon l'équation Vs=Sa/10, Vs étant le volume de SBF et Sa étant la surface de l'échantillon (mm²).

Les temps d'incubation sont de 1 jour (D1), 3 jours (D3) et de 7 jours (D7).

Des échantillons correspondant à l'EX4 sans bioverre sont également mis en œuvre comme échantillons tests.

Les paramètres d'incubation sont de 80 tours/min, et la température de la solution de SBF est conservée à 37°C dans l'agitateur. La solution de SBF est renouvelée tous les 48 heures.

### E- Caractérisation des échantillons des exemples 1 à 4 (EX1 à EX4) testés

L'analyse morphologique et structurelle des échantillons et la caractérisation de surface sont effectuées par microscope à balayage électronique (SEM, Hitachi flex 1000) opérant à un voltage de 5 kV. Les échantillons testés (EX1 à EX4) sont rincés à l'eau distillée, et séchés. Tous les échantillons sont ensuite pulvérisés d'une enduction fine de chrome (5nm d'épaisseur) sur leurs surfaces extérieures. La méthodologie d'évaluation consiste à analyser 3 sites différents de la surface extérieure du membre de couverture et de la surface intérieure du membre de couverture, ainsi que la surface extérieure de l'âme longiligne.

La caractérisation chimique des échantillons en surface est réalisée par spectroscopie de rayons X à dispersion d'énergie (EDS). L'acquisition des motifs de diffraction est réalisée sur le diffractomètre PAnalytical X'Pert Pro MRD, avec une anode (Cu) en tant que source de rayon X (longueur d'onde caractéristique de 1,5418 ansgtröm). Les diffractogrammes ont été obtenus dans l'intervalle de 5 à 70° (theta) avec un détecteur (X'Celelerator^{®}, PANalytical) fourni avec une fente anti-divergence de 0,5° et 1° et masque de 10 mm.

### F- Etude de cytotoxicité in vitro

Une étude de cytotoxicité a été effectuée sur les exemples EX1 à EX4 selon la norme ISO 10993-5, datant de 2009. Cette dernière s'est avérée positive, c'est-à-dire que le taux de survie des cellules vivantes est satisfaisant.

### G- Commentaires

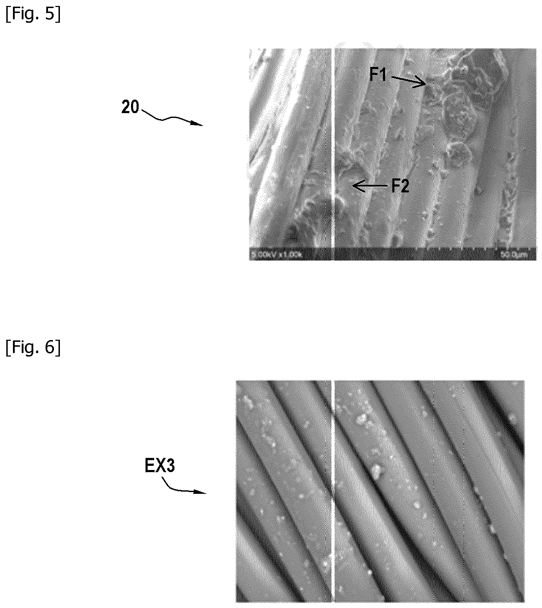

La figure 5 représente une photographie par SEM de la surface extérieure de l'âme longiligne 20 obtenue au point B. Les flèches F1 et F2 pointent la formation de cristaux de bioverre au terme de 7 jours d'incubation dans le SBF.

On remarque aux termes de 1 jour et de 3 jours d'incubation, l'absence de bio-minéralisation selon la surface extérieure de l'âme longiligne 20, selon la surface intérieure ou encore la surface extérieure du membre de couverture des exemples 1 à 4.

Après 7 jours d'incubation, on remarque une légère bio-minéralisation sur la surface extérieure des membres de couverture des exemples 3 et 4 à proximité des orifices traversants.

On remarque une bio-minéralisation importante sur toutes les surfaces intérieures des membres de couverture des exemples 1 à 4 au terme de 7 jours d'incubation.

En particulier, on remarque que de plus nombreux cristaux de bioverre sont présents sur la surface extérieure du membre de couverture de l'exemple 4 représenté à la figure 8 que selon la surface extérieure du membre de couverture de l'exemple 3 représenté à la figure 6. Cependant, les orifices traversants des exemples 3 et 4 ont permis avantageusement la bio-minéralisation de la surface extérieure du membre de couverture.

Tel que cela est visible sur la figure 7, le calcium et le phosphore sont présents abondamment sur la surface extérieure de l'âme longiligne (IB) et sur la surface intérieure du membre de couverture (IF) de l'exemple 3. On retrouve également encore la présence de calcium et de phosphore selon la surface extérieure du membre de couverture (EF) de l'exemple 3.

En calculant le ratio molaire entre le calcium et le phosphore, on retrouve un ratio Ca/P au terme de 7 jours d'incubation de 1,125 selon la surface extérieure du membre de couverture de l'exemple 3, et de 1,75 selon la surface extérieure du membre de couverture de l'exemple 4.

Le ratio molaire de l'hydroxyapatite est estimé comme étant de 1,67.

Enfin, davantage de bio-minéralisation est observé au terme de 14 jours ou 21 jours d'incubation pour les exemples 1 à 4.

Avantageusement, le dispositif implantable selon l'invention assure la fonction mécanique de tendon ou de ligament mais permet également la libération progressive d'agent(s) fonctionnel(s), en particulier permet la formation d'hydroxyapatite sur la surface extérieure du membre de couverture, en particulier dès 7 jours pour les exemples 3 et 4.

## Revendications

1. Dispositif implantable, en particulier pour la reconstruction d'un ligament croisé antérieur ou d'un tendon, comprenant une âme longiligne (20) et un membre de couverture longiligne creux comprenant un volume intérieur recevant au moins en partie ladite âme longiligne, le membre de couverture longiligne creux comprend des orifices traversants en communication fluidique avec ledit volume intérieur, **caractérisé en ce que** ladite âme longiligne comprend une matrice polymère bio-érodable en milieu aqueux et un ou plusieurs agent(s) fonctionnel(s), **en ce que** l'âme longiligne (20) comprend une âme longiligne textile revêtue au moins en partie de ladite matrice polymère bio-érodable dans laquelle ledit ou lesdits agent(s) fonctionnel(s) est/sont dispersé(s), **en ce que** le membre de couverture longiligne creux est une tresse creuse, et **en ce que** le ou les agent(s) fonctionnel(s) comprend/comprennent un ou plusieurs agent(s) ostéo-inducteur(s) et/ou ostéo-conducteur(s) et/ou le ou les agent(s) fonctionnel(s) sont choisi(s) parmi : les anti-inflammatoires, les antibiotiques, les antibactériens, les antalgiques, et un mélange de ces derniers.

2. Dispositif implantable selon la revendication 1, **caractérisé en ce que** le ou les agent(s) ostéo-inducteur(s) et/ou ostéo-conducteur(s) est/sont choisi(s) parmi : les bioverres, les biocéramiques à base de phosphate de calcium, notamment les hydroxyapatites, et le phosphate tricalcique, et un mélange de ces derniers.

3. Dispositif implantable selon l'une ou l'autre des revendications 1 et 2, **caractérisé en ce que** l'âme longiligne (20) comprend une tresse.

4. Dispositif implantable selon l'une quelconque des revendications 1 à 3, **caractérisé en ce que** l'âme longiligne textile comprend une âme auxiliaire comprenant un ou plusieurs fils, et une couverture auxiliaire textile tressée disposée autour de ladite âme auxiliaire.

5. Dispositif implantable selon l'une quelconque des revendications 1 à 4, **caractérisé en ce que** la matrice polymère bio-érodable comprend un polymère, ou plusieurs polymères mélangé(s), déshydraté(s) et apte(s) à former un gel soluble en milieu aqueux, en particulier bio-érodable(s).

6. Dispositif implantable selon l'une quelconque des revendications 1 à 5, **caractérisé en ce que** la matrice polymère bio-érodable comprend un ou plusieurs polymère(s) bio-érodable(s) choisi(s) parmi les polymères suivants : des (co)polymères de cyclodextrine(s) et/ou de dérivé(s) de cyclodextrine(s) et/ou de complexe(s) d'inclusion de cyclodextrine(s) et/ou de dérivé(s) de cyclodextrine(s) solubles ; les polymères de collagène ; les polymères dérivés de l'acide hyaluronique ; les polymères de carboxyméthylcellulose ou dérivés de la carboxyméthylcellulose ; les polymères vinyliques tels que les polymères de polyvinylpyrrolidone (PVP) ou les polymères de polyvinylpolypyrrolidone (crospovidone) ; les polymères de polyéthylène glycol ; les polymères d'acide lactique ; les copolymères d'acide lactique, et de polyéthylèneglycol (PLLA/PEG) ; les copolymères d'acide lactique, d'acide glycolique et de polyéthylèneglycol (PLGA/PEG) ; les polymères d'alcools polyvinylique (PVA) ; et les polymères de polyacrylates tel que le polyhydroxyéthylméthacrylate (PHEM) ; et un mélange de ces derniers.

7. Dispositif implantable selon l'une quelconque des revendications 1 à 6, **caractérisé en ce que** la matrice polymère bio-érodable comprend un polymère choisi parmi : les polymères de polyvinylpyrrolidone (PVP) ; les polymères d'alcool polyvinylique ; les (co)polymères de cyclodextrine(s) et/ou de dérivé(s) de cyclodextrine(s) et/ou de complexe(s) d'inclusion de cyclodextrine(s) et/ou de dérivé(s) de cyclodextrine(s) solubles.

8. Dispositif implantable selon l'une quelconque des revendications 1 à 7, **caractérisé en ce que** la matrice polymère bio-érodable comprend un ou plusieurs polymère(s) bio-érodable(s), et **en ce que** le ratio massique agent(s) fonctionnel(s) / polymère(s) bio-érodable(s) dans la matrice polymère, en particulier le ratio massique charge(s) ostéo-inductrice(s) et/ou ostéo-inducteur(s) / polymère(s) bio-érodable(s) dans la matrice polymère, est supérieur ou égal à 0,2 et inférieur ou égal à 1,8 ; de préférence supérieur ou égal à 0,2 et inférieur ou égal à 1.

9. Dispositif implantable selon l'une quelconque des revendications 1 à 8, **caractérisé en ce que** le membre de couverture longiligne creux comprend une tresse comprenant entre 8 et 30 mèches tressées, et **en ce que** chaque mèche tressée comprend entre 2 et 8 fils, de préférence lesdits fils ont chacun un titrage compris entre 80 dtex et 200 dtex.

10. Dispositif implantable selon l'une quelconque des revendications 1 à 9, **caractérisé en ce que** le membre de couverture longiligne creux comprend entre 30 et 120 fils, de préférence entre 30 fils et 80 fils, notamment lesdits fils ne sont pas biorésorbables.

11. Dispositif implantable selon l'une quelconque des revendications 1 à 10, **caractérisé en ce que** la fraction massique de la matrice polymère bio-érodable comprenant le ou les agent(s) fonctionnel(s) dans ledit dispositif implantable est supérieure ou égale à 5% et inférieure ou égale à 25%, de préférence supérieure ou égale à 10% et inférieure ou égale à 20%.

12. Dispositif implantable selon l'une quelconque des revendications 1 à 11, **caractérisé en ce que** la fraction massique de l'âme longiligne, exempte de la matrice polymère et dudit ou desdits agent(s) fonctionnel(s), et du membre de couverture longiligne creux mesurée par rapport à la masse totale du dispositif implantable est supérieure ou égale à 50%, de préférence supérieure ou égale à 75%.

13. Dispositif implantable selon l'une quelconque des revendications 1 à 12, **caractérisé en ce que** le membre de couverture longiligne creux est une tresse creuse avec un angle de tressage sensiblement constant supérieur ou égal à 2 mm et inférieur ou égal à 15 mm, de préférence supérieur ou égal à 4 mm et inférieur ou égal à 10 mm.

14. Procédé de fabrication d'un dispositif implantable, notamment selon l'une quelconque des revendications 1 à 13, **caractérisé en ce qu'**il comprend :
a) une étape d'application d'un revêtement polymérique bio-érodable, comprenant un ou plusieurs agent(s) fonctionnel(s), au moins en partie sur une âme longiligne textile,
b) une étape de surtressage d'un membre de couverture longiligne creux autour au moins en partie de ladite âme longiligne textile revêtue au moins en partie ;
c) une étape de récupération d'un dispositif implantable comprenant une âme longiligne comprenant une âme longiligne textile et un membre de couverture longiligne creux comprenant un volume intérieur recevant au moins en partie ladite âme longiligne, ladite âme longiligne comprend une matrice polymère bio-érodable en milieu aqueux revêtant au moins en partie ladite âme longiligne textile, et un ou plusieurs agent(s) fonctionnel(s) dispersé(s) dans la matrice polymère bio-érodable, **en ce que** le membre de couverture longiligne comprend des orifices traversants en communication fluidique avec ledit volume intérieur, et **en ce que** le ou les agent(s) fonctionnel(s) comprend/comprennent un ou plusieurs agent(s) ostéo-inducteur(s) et/ou ostéo-conducteur(s) et/ou le ou les agent(s) fonctionnel(s) sont choisi(s) parmi : les anti-inflammatoires, les antibiotiques, les antibactériens, les antalgiques, et un mélange de ces derniers.

15. Procédé de fabrication selon la revendication 14, **caractérisé en ce que** l'étape b) est une étape de tressage d'une tresse creuse sur un dispositif de tressage comprenant un nombre total de fuseaux supérieur ou égal à 12 et inférieur ou égal à 30, et **en ce que** le nombre de fuseaux vides (36,38,46,48,62,64,66,68,82,84,86,88) est supérieur ou égal à 10% et inférieur ou égal à 60% du nombre total de fuseaux, en particulier supérieur ou égal à 20% et inférieur ou égal à 60% du nombre total de fuseaux.

16. Procédé de fabrication selon l'une ou l'autre des revendications 14 et 15, **caractérisé en ce que** l'étape b) est une étape de tressage d'une tresse creuse sur un dispositif de tressage comprenant des fuseaux pleins (32,34,42,44,52,54,56,58,72,74,76,78) et des fuseaux vides (36,38,46,48,62,64,66,68,82,84,86,88), et **en ce que** les fuseaux pleins (52,54,56,58,72,74, 76,78) sont répartis dans au moins trois groupes de fuseaux pleins dont chaque groupe comprend des fuseaux pleins voisins, et **en ce qu'**un groupe de fuseaux pleins est séparé d'un autre groupe de fuseaux pleins par au moins un fuseau vide (62,64,66,68,82,84,86,88), de préférence par au moins deux ou trois fuseaux vides.

17. Procédé de fabrication selon la revendication 16, **caractérisé en ce que** le dispositif de tressage comprend trois ou quatre groupes de trois ou quatre fuseaux pleins voisins et trois ou quatre groupes de fuseaux vides voisins, un groupe de fuseaux pleins voisins étant disposé entre deux groupes de fuseaux vides voisins.

18. Procédé de fabrication selon l'une quelconque des revendications 14 à 17, **caractérisé en ce que** l'étape b) est une étape de tressage d'une tresse creuse avec un angle de tressage sensiblement constant supérieur ou égal à 2 mm et inférieur ou égal à 15 mm, de préférence supérieur ou égal à 4 mm et inférieur ou égal à 10 mm.

## Patentansprüche

1. Implantierbare Vorrichtung, im Speziellen für die Rekonstruktion eines vorderen Kreuzbandes oder einer Sehne, umfassend eine längliche Seele (20) und ein längliches hohles Abdeckelement, das ein Innenvolumen umfasst, welches zumindest zum Teil die längliche Seele aufnimmt, wobei das längliche hohle Abdeckelement Durchgangsöffnungen in Fluidverbindung mit dem Innenvolumen umfasst, **dadurch gekennzeichnet, dass** die längliche Seele eine in wässrigem Milieu biologisch erodierbare Polymermatrix und ein oder mehrere funktionelle Mittel umfasst, dass die längliche Seele (20) eine längliche Textilseele umfasst, die zumindest zum Teil von der biologisch erodierbaren Polymermatrix bedeckt ist, in welcher das oder die funktionellen Mittel dispergiert ist/sind, dass das längliche hohle Abdeckelement ein hohles Geflecht ist, und dass das oder die funktionellen Mittel ein oder mehrere osteoinduktive und/oder osteokonduktive Mittel umfasst/umfassen und/oder das oder die funktionellen Mittel aus: den entzündungshemmenden Mitteln, Antibiotika, den antibakteriellen Mitteln, den Schmerzmitteln und einer Mischung davon ausgewählt ist/sind.

2. Implantierbare Vorrichtung nach Anspruch 1, **dadurch gekennzeichnet, dass** das oder die osteoinduktiven und/oder osteokonduktiven Mittel aus: den Biogläsern, den Biokeramiken auf Basis von Calciumphosphat, insbesondere den Hydroxyapatiten, und Tricalciumphosphat, und einer Mischung davon ausgewählt ist/sind.

3. Implantierbare Vorrichtung nach einem von Anspruch 1 und 2, **dadurch gekennzeichnet, dass** die längliche Seele (20) ein Geflecht umfasst.

4. Implantierbare Vorrichtung nach einem der Ansprüche 1 bis 3, **dadurch gekennzeichnet, dass** die längliche Textilseele eine Zusatzseele umfassend einen oder mehrere Fäden und eine geflochtene Zusatz-Textilabdeckung umfasst, die um die Zusatzseele herum angeordnet ist.

5. Implantierbare Vorrichtung nach einem der Ansprüche 1 bis 4, **dadurch gekennzeichnet, dass** die biologisch erodierbare Polymermatrix ein Polymer oder mehrere Polymere umfasst, das/die gemischt, dehydriert und dazu geeignet, ein in wässrigem Milieu lösliches Gel zu bilden, und insbesondere biologisch erodierbar ist/sind.

6. Implantierbare Vorrichtung nach einem der Ansprüche 1 bis 5, **dadurch gekennzeichnet, dass** die biologisch erodierbare Polymermatrix ein oder mehrere biologisch erodierbare Polymere umfasst, das/die aus den folgenden Polymeren ausgewählt ist/sind: den löslichen (Co)polymeren von Cyclodextrin(en) und/oder von Derivaten von Cyclodextrin(en) und/oder von Inklusionskomplex(en) von Cyclodextrin(en) und/oder von Derivat(en) von Cyclodextrin(en), den Kollagen-Polymeren, den von Hyaluronsäure abgeleiteten Polymeren, den Polymeren von Carboxymethylcellulose oder Derivaten von Carboxymethylcellulose, den Vinylpolymeren wie etwa den Polyvinylpyrrolidon-Polymeren (PVP) oder den Polyvinylpolypyrrolidon-Polymeren (Crospovidon), den Polymeren von Polyethylenglykol, den Polymeren von Milchsäure, den Copolymeren von Milchsäure und Polyethylenglykol (PLLA/PEG), den Copolymeren von Milchsäure, Glykolsäure und Polyethylenglykol (PLGA/PEG), den Polymeren von Polyvinylalkoholen (PVA), und den Polymeren von Polyacrylaten wie etwa Polyhydroxyethylmethacrylat (PHEM), und einer Mischung davon.

7. Implantierbare Vorrichtung nach einem der Ansprüche 1 bis 6, **dadurch gekennzeichnet, dass** die biologisch erodierbare Polymermatrix ein Polymer umfasst, das ausgewählt ist aus: den Polyvinylpyrrolidon-Polymeren (PVP), den Polymeren von Polyvinylalkoholen, den löslichen (Co)polymeren von Cyclodextrin(en) und/oder von Derivaten von Cyclodextrin(en) und/oder von Inklusionskomplex(en) von Cyclodextrin(en) und/oder von Derivat(en) von Cyclodextrin(en).

8. Implantierbare Vorrichtung nach einem der Ansprüche 1 bis 7, **dadurch gekennzeichnet, dass** die biologisch erodierbare Polymermatrix ein oder mehrere biologisch erodierbare Polymere umfasst, und dass das Massenverhältnis funktionelle(s) Mittel / biologisch erodierbare(s) Polymer(e) in der Polymermatrix, insbesondere das Massenverhältnis osteodinduktive und/oder osteokonduktive Ladung(en) / biologisch erodierbare(s) Polymer(e) in der Polymermatrix, größer oder gleich 0,2 und kleiner oder gleich 1,8 ist, vorzugsweise größer oder gleich 0, 2 und kleiner oder gleich 1.

9. Implantierbare Vorrichtung nach einem der Ansprüche 1 bis 8, **dadurch gekennzeichnet, dass** das längliche hohle Abdeckelement ein Geflecht umfasst, das zwischen 8 und 30 verflochtene Stränge umfasst, und dass jeder verflochtene Strang zwischen 2 und 8 Fäden umfasst, wobei die Fäden vorzugsweise jeweils einen Titer zwischen 80 dtex und 200 dtex aufweisen.

10. Implantierbare Vorrichtung nach einem der Ansprüche 1 bis 9, **dadurch gekennzeichnet, dass** das längliche hohle Abdeckelement zwischen 30 und 120 Fäden, vorzugsweise zwischen 30 Fäden und 80 Fäden umfasst, wobei insbesondere die Fäden nicht biologisch resorbierbar sind.

11. Implantierbare Vorrichtung nach einem der Ansprüche 1 bis 10, **dadurch gekennzeichnet, dass** der Massenanteil der biologisch erodierbaren Polymermatrix, welche das oder die funktionellen Mittel umfasst, in der implantierbaren Vorrichtung größer oder gleich 5 % und kleiner oder gleich 25 % ist, vorzugsweise größer oder gleich 10 % und kleiner oder gleich 20 %.

12. Implantierbare Vorrichtung nach einem der Ansprüche 1 bis 11, **dadurch gekennzeichnet, dass** der Massenanteil der länglichen Seele, ohne die Polymermatrix und das oder die funktionellen Mittel, und des länglichen hohlen Abdeckelements, gemessen im Verhältnis zur Gesamtmasse der implantierbaren Vorrichtung größer oder gleich 50 % ist, vorzugsweise größer oder gleich 75 %.

13. Implantierbare Vorrichtung nach einem der Ansprüche 1 bis 12, **dadurch gekennzeichnet, dass** das längliche hohle Abdeckelement ein hohles Geflecht mit einem im Wesentlichen konstanten Flechtwinkel größer oder gleich 2 mm und kleiner oder gleich 15 mm ist, vorzugsweise größer oder gleich 4 mm und kleiner oder gleich 10 mm.

14. Verfahren zur Herstellung einer implantierbaren Vorrichtung, insbesondere nach einem der Ansprüche 1 bis 13, **dadurch gekennzeichnet, dass** es umfasst:
a) einen Schritt des Aufbringens einer biologisch erodierbaren Polymerbeschichtung, umfassend ein oder mehrere funktionelle Mittel, zumindest zum Teil auf eine längliche Textilseele,
b) einen Schritt des Überflechtens eines hohlen, länglichen Abdeckelements zumindest zum Teil um die längliche zumindest zum Teil abgedeckte Textilseele herum,
c) einen Schritt des Erhaltens einer implantierbaren Vorrichtung, die eine längliche Seele umfassend eine längliche Textilseele und ein längliches hohles Abdeckelement umfasst, das ein Innenvolumen umfasst, welches zumindest zum Teil die längliche Seele aufnimmt, wobei die längliche Seele eine in wässrigem Milieu biologisch erodierbare Polymermatrix, die zumindest zum Teil die längliche Textilseele abdeckt, und ein oder mehrere funktionelle Mittel umfasst, die in der biologisch erodierbaren Polymermatrix dispergiert ist/sind, wobei das längliche hohle Abdeckelement Durchgangsöffnungen in Fluidverbindung mit dem Innenvolumen umfasst, und wobei das oder die funktionellen Mittel ein oder mehrere osteoinduktive und/oder osteokonduktive Mittel umfasst/umfassen und/oder das oder die funktionellen Mittel aus: den entzündungshemmenden Mitteln, Antibiotika, den antibakteriellen Mitteln, den Schmerzmitteln und einer Mischung davon ausgewählt ist/sind.

15. Herstellungsverfahren nach Anspruch 14, **dadurch gekennzeichnet, dass** der Schritt b) ein Schritt des Flechtens eines hohlen Geflechts auf einer Flechtvorrichtung ist, welche eine Gesamtzahl von Flechtklöppeln größer oder gleich 12 und kleiner oder gleich 30 umfasst, und dass die Anzahl von leeren Klöppeln (36, 38, 46, 48, 62, 64, 66, 68, 82, 84, 86, 88) größer oder gleich 10 % und kleiner oder gleich 60 % der Gesamtzahl von Klöppeln ist, insbesondere größer oder gleich 20 % und kleiner oder gleich 60 % der Gesamtzahl von Klöppeln.

16. Herstellungsverfahren nach einem von Anspruch 14 und Anspruch 15, **dadurch gekennzeichnet, dass** der Schritt b) ein Schritt des Flechtens eines hohlen Geflechts auf einer Flechtvorrichtung ist, die volle Klöppel (32, 34, 42, 44, 52, 54, 56, 58, 72, 74, 76, 78) und leere Klöppel (36, 38, 46, 48, 62, 64, 66, 68, 82, 84, 86, 88) umfasst, und dass die vollen Klöppel (52, 54, 56, 58, 72, 74, 76, 78) in zumindest drei Gruppen von vollen Klöppeln unterteilt sind, wovon jede Gruppe benachbarte volle Klöppel umfasst, und dass eine Gruppe von vollen Klöppeln von einer weiteren Gruppe von vollen Klöppeln durch zumindest einen leeren Klöppel (62, 64, 66, 68, 82, 84, 86, 88) getrennt ist, vorzugsweise durch zumindest zwei oder drei leere Klöppel.

17. Herstellungsverfahren nach Anspruch 16, **dadurch gekennzeichnet, dass** die Flechtvorrichtung drei oder vier Gruppen von drei oder vier benachbarten vollen Klöppeln und drei oder vier Gruppen von benachbarten leeren Klöppeln umfasst, wobei eine Gruppe von benachbarten vollen Klöppeln zwischen zwei Gruppen von benachbarten leeren Klöppeln angeordnet ist.

18. Herstellungsverfahren nach einem der Ansprüche 14 bis 17, **dadurch gekennzeichnet, dass** der Schritt b) ein Schritt des Flechtens eines hohlen Geflechts mit einem im Wesentlichen konstanten Flechtwinkel von größer oder gleich 2 mm und kleiner oder gleich 15 mm ist, vorzugsweise größer oder gleich 4 mm und kleiner oder gleich 10 mm.

## Claims

1. An implantable device, in particular for the reconstruction of an anterior cruciate ligament or tendon, comprising an elongate core (20) and a hollow elongate covering member comprising an inside volume at least partially receiving said elongate core, the hollow elongate covering member comprises through-holes in fluid communication with said inside volume, **characterized in that** said elongate core comprises a polymer matrix bio-erodible in an aqueous medium and one or more functional agent(s), **in that** the elongate core (20) comprises a textile elongate core at least partially coated with the bio-erodible polymer matrix within which the functional agent(s) are dispersed, **in that** the hollow elongate covering member is a hollow braid, and **in that** the functional agent(s) comprise(s) one or more osteoinductive and/or osteoconductive agents and/or the functional agent(s) are chosen from among: anti-inflammatories, antibiotics, antibacterials, analgesics and a mixture thereof.

2. The implantable device according to claim 1, **characterized in that** the osteoinductive and/or osteoconductive agent(s) are chosen from among: bioglass, calcium phosphate-based bioceramics, in particular hydroxyapatites, and tricalcium phosphate, and a mixture thereof.

3. The implantable device according to either one of claims 1 and 2, **characterized in that** the elongate core (20) comprises a braid.

4. The implantable device according to any of claims 1 to 3, **characterized in that** the textile elongate core comprises an auxiliary core comprising one or more yarn(s), and an auxiliary textile covering braided around said auxiliary core.

5. The implantable device according to any of claims 1 to 4, **characterized in that** the bio-erodible polymer matrix comprises a polymer, or several mixed polymers, that are dehydrated and able to form a soluble gel in an aqueous medium, in particular bio-erodible.

6. The implantable device according to any of claims 1 to 5, **characterized in that** the bio-erodible polymer matrix comprises one or more bio-erodible polymers chosen from among the following polymers: (co)polymers of cyclodextrin(s) and/or of derivative(s) of cyclodextrin(s) and/or of inclusion complex(es) of cyclodextrin(s) and/or of derivatives of soluble cyclodextrin(s); collagen polymers; polymers derived from hyaluronic acid; polymers of carboxymethylcellulose or derivatives of carboxymethylcellulose; vinyl polymers such as polyvinylpyrrolidone polymers (PVP) or polyvinylpolypyrrolidone polymers (crospovidone); polyethylene glycol polymers; lactic acid polymers; copolymers of lactic acid and polyethyleneglycol (PLLA/PEG); copolymers of lactic acid, glycolic acid and polyethyleneglycol (PLGA/PEG); polymers of polyvinyl alcohols (PVA); and polymers of polyacrylates such as polyhydroxyethylmethacylate (PHEM); and a mixture thereof.

7. The implantable device according to any of claims 1 to 6, **characterized in that** the bio-erodible polymer matrix comprises a polymer chosen from among: polyvinylpyrrolidone polymers (PVP); polyvinyl alcohol polymers; (co)polymers of cyclodextrin(s) and/or derivative(s) of cyclodextrin(s) and/or of inclusion complex(es) of cyclodextrin(s) and/or of soluble cyclodextrin derivative(s).

8. The implantable device according to any of claims 1 to 7, **characterized in that** the bio-erodible polymer matrix comprises one or more bio-erodible polymers, and **in that** the weight ratio of functional agent(s)/bio-erodible polymer(s) in the polymer matrix, in particular the weight ratio of osteoinductive and/or osteoconductive fillers/ bio-erodible polymer(s) in the polymer matrix, is higher than or equal to 0.2 and lower than or equal to 1.8; preferably higher than or equal to 0.2 and lower than or equal to 1.

9. The implantable device according to any of claims 1 to 8, **characterized in that** the hollow elongate covering member comprises a braid comprising between 8 and 30 braided threads, and **in that** each braided thread comprises between 2 and 8 yarns, preferably said yarns each have a titer of between 80 dtex and 200 dtex.

10. The implantable device according to any of claims 1 to 9, **characterized in that** the hollow elongate covering member comprises between 30 and 120 yarns, preferably between 30 yarns and 80 yarns, in particular said yarns are not bioresorbable.

11. The implantable device according to any of claims 1 to 10, **characterized in that** the mass fraction of the bio-erodible polymer matrix comprising the functional agent(s) in said implantable device is higher than or equal to 5 % and lower than or equal to 25%, preferably higher than or equal to 10% and lower than or equal to 20%.

12. The implantable device according to any of claims 1 to 11, **characterized in that** the mass fraction of the elongate core, free of the polymer matrix and of said functional agent(s), and of said hollow elongate covering member, measured relative to the total mass of the implantable device, is higher than or equal to 50%, preferably higher than or equal to 75%.

13. The implantable device according to any of claims 1 to 12, **characterized in that** the hollow elongate covering member is a hollow braid with a substantially constant braiding angle greater than or equal to 2 mm and smaller than or equal to 15 mm, preferably greater than or equal to 4 mm and smaller than or equal to 10 mm.

14. A method for manufacturing an implantable device, in particular according to any of claims 1 to 13, **characterized in that** it comprises:
a) a step to apply a bio-erodible polymer coating, comprising one or more functional agents, at least in part onto an elongate textile core,
b) an over-braiding step of a hollow elongate covering member around at least in part of said at least partially coated textile elongate core;
c) a step to recover an implantable device comprising an elongate core comprising a textile elongate core and a hollow elongate covering member comprising an inside volume at least partially receiving said elongate core, said elongate core comprises a polymer matrix bio-erodible in an aqueous medium coating at least partially said textile elongate core, and one or more functional agents dispersed in the bio-erodible polymer matrix, **in that** the covering member comprises through-holes in fluid communication with said inside volume, and **in that** the functional agent(s) comprise one or more osteoinductive and/or osteoconductive agents and/or the functional agent(s) are chosen from among: anti-inflammatories, antibiotics, antibacterials, analgesics and a mixture thereof.

15. The manufacturing method according to claim 14, **characterized in that** step b) is a step of braiding a hollow braid on a braiding device comprising a total number of spindles higher than or equal to 12 and lower than or equal to 30, and **in that** the number of empty spindles (36,38,46,48,62,64,66,68,82,84,86,88) is higher than or equal to 10% and lower than or equal to 60% of the total number of spindles, in particular higher than or equal to 20% and lower than or equal to 60% of the total number of spindles.

16. The manufacturing method according to either one of claims 14 and 15, **characterized in that** step b) is a braiding step of a hollow braid on a braiding device comprising filled spindles (32,34,42,44,52,54,56,58,72,74,76,78) and empty spindles (36,38,46,48,62,64,66,68,82,84,86,88), and **in that** the filled spindles (52,54,56,58,72,74,76,78) are distributed in at least three groups of filled spindles in which each group comprises adjacent filled spindles, and **in that** a group of filled spindles is separated from another group of filled spindles by at least one empty spindle (62,64,66,68,82,84,86,88), preferably by at least two or three empty spindles.

17. The manufacturing method according to claim 16, **characterized in that** the braiding device comprises three or four groups of three or four adjacent filed spindles, and three or four groups of adjacent empty spindles, a group of adjacent filled spindles being arranged between two groups of adjacent empty spindles.

18. The manufacturing method according to any of claims 14 to 17, **characterized in that** step b) is a braiding step of a hollow braid at a substantially constant braiding angle greater than or equal to 2 mm and smaller than or equal to 15 mm, preferably greater than or equal to 4 mm and smaller than or equal to 10 mm.
